# EUROPÄISCHE PATENTSCHRIFT

(11) **EP 2 664 300 B1**
(45) Veröffentlichungstag und Bekanntmachung des Hinweises auf die Patenterteilung: **28.10.2020**
(21) Anmeldenummer: 12167842.9
(22) Anmeldetag: 14.05.2012
(51) Int. Cl.: A61F 2/14, A61F 2/00

(54) **Intracorneallinse**
Intracorneal lens
Lentille intracornéenne

(43) Veröffentlichungstag der Anmeldung: 20.11.2013
(73) Patentinhaber: Presbia Ireland Limited, Dublin 2 (IE)
(72) Erfinder: Berner, Werner, 5018 Erlinsbach (CH)
(74) Vertreter: Ahmad, Sheikh Shakeel

(56) Entgegenhaltungen:
- WO-A1-2009/075685
- WO-A1-2011/055066
- WO-A1-2011/069907
- US-A- 5 196 026
- US-A1- 2004 088 049
- US-A1- 2005 246 015

## Beschreibung

Die vorliegende Erfindung betrifft eine Intracorneallinse zur Korrektur von Sehschwächen wie beispielsweise Presbyopie. Intracorneallinsen werden zur Korrektur von Sehschwächen herangezogen. Im Gegensatz zu Kontaktlinsen, welche auf der Augenoberfläche aufgebracht sind, und zu Intraocularlinsen, welche in eine Augenkammer implantiert werden, setzt man Intracorneallinsen in eine in der Hornhaut (Cornea) geschaffene Tasche ein. Intracorneallinsen unterscheiden sich signifikant von Kontaktlinsen oder Intraocularlinsen, beispielsweise in ihrer Grösse, dem Fehlen von bei Intraocularlinsen erforderlichen Halteelementen (haptischen Elementen) sowie in ihren optischen Eigenschaften.

Intracorneallinsen sind aus dem Stand der Technik bekannt. Beispielhaft sei auf die WO 2009/075685, die US-5,628,794, die US-5,123,921 oder die EP-1 001 720 B1 verwiesen. Weitere Intracorneallinsen sind aus der US 2004/0088049 A1 oder der WO 2011/055066 A1 bekannt.

In der WO 2009/075685 ist eine Intracorneallinse beschrieben, welche ein zentrales Loch aufweist. Das zentrale Loch ist konzentrisch mit der optischen Achse der Linse und weist eine derartige Grösse und Form auf, dass die optischen Eigenschaften der Linse nicht beeinträchtigt werden, gleichzeitig aber das Loch zu einer prazisen Positionierung der Linse in der Hornhauttasche verwendet werden kann.

Es hat sich jedoch gezeigt, dass diese Linsen noch nicht die optimale Korrektur des Sehvermögens beim Menschen bewirken.

Es bestand daher Bedarf nach einer Intracorneallinse, mit welcher das Sehvermögen von Probanden mit einer Sehschwäche wie Presbyopie noch besser wiederhergestellt werden kann. Erfindungsgemäss wurde die vorstehende Aufgabe gelöst durch eine Intracorneallinse, umfassend einen kreisförmigen Grundkörper mit einer konvexen vorderen Oberfläche und einer konkaven hinteren Oberfläche, dadurch gekennzeichnet, dass die konvexe vordere Oberfläche einen einzigen gleichmässigen Krümmungsradius Rcv und die konkave hintere Oberfläche einen Krümmungsradius Rcci aufweist, wobei der Krümmungsradius Rcci der konkaven hinteren Oberfläche um 0,1 mm bis 2 mm, vorzugsweise 0,2 bis 1,5 mm, insbesondere bevorzugt 0,5 bis 1 mm grösser ist als der mittleren Radius der Cornea, welcher abhängig vom Individuum in einem Bereich von 7,4 mm bis 8,1 mm liegt, wobei die konkave hintere Oberfläche mindestens zwei, vorzugsweise genau zwei Zonen mit unterschiedlichen Krümmungsradien aufweist.

Es hat sich überraschend gezeigt, dass eine deutliche Verbesserung der Sehkorrektur durch Intracorneallinsen erreicht werden kann, wenn der Krümmungsradius Rcci der konkaven hinteren Oberfläche der Linse den mittleren Radius der Cornea etwas überschreitet. Dadurch schmiegt sich die erfindungsgemässe Linse wegen ihrer geringen Eigensteifigkeit auf einfache Weise gefaltet, an die Hornhaut an und übt weniger Kräfte auf die Hornhaut aus. Gleichzeitig wurde aber gefunden, dass der Krümmungsradius Rcci der konkaven hinteren Oberfläche der Linse den mittleren Radius der Cornea nur um einen bestimmten geringen Wert überschreiten darf, da es sonst an den äusseren Rändern der Linse zur Bildung von Falten in der Cornea und damit verbundenen negativen optischen Effekten kommt.

Gemäss der vorliegenden Erfindung ist der Krümmungsradius Rcci der konkaven hinteren Oberfläche um 0,1 mm bis 2 mm, vorzugsweise 0,2 bis 1,5 mm, insbesondere bevorzugt 0,5 bis 1 mm grösser als der mittlere Radius der Cornea.

Weiterhin wurde gefunden, dass der Fall der Bereitstellung von Linsenzonen mit unterschiedlichen Krümmungsradien dadurch realisiert wird, dass die unterschiedlichen Krümmungsradien auf der konkaven hinteren Oberfläche der Linse angeordnet werden, während die konvexe vordere Oberfläche der Linse einen einzigen gleichmässigen Krümmungsradius Rcv aufweist. Eine durch unterschiedliche Krümmungsradien der implantierten Intracorneallinse hervorgerufene Veränderung der Krümmung der Cornea hat an der Aussenseite der Cornea (Grenzfläche zur Umgebung) aufgrund des signifikanten Unterschieds der Brechungsindices der Cornea und der Luft einen erheblichen Einfluss auf die optischen Eigenschaften des korrigierten Auges, während eine analoge Veränderung der Krümmung der Cornea an der Grenzfläche zum Augeninneren einen etwa um den Faktor 5 geringeren Einfluss hat (geringerer Unterschied der Brechungsindices der Cornea und der Augenflüssigkeit).

Durch die vorstehenden Massnahmen können mit der erfindungsgemässen Intracorneallinse deutlich bessere und präzisere Korrekturen der Sehschärfe erreicht werden als mit herkömmliche Intracorneallinsen. Mit der erfindungsgemässen Intracorneallinse können Sehschwächen wie Myopie (Kurzsichtigkeit), Hyperopie (Weitsichtigkeit), Presbyopie (altersbedingtes Nachlassen der Akkomodationsfähigkeit des Auges) oder eine Kombination dieser Sehschwächen korrigiert werden. Bei einer entsprechenden, dem Fachmann bekannten Optimierung der Flächen der Linse ist es auch möglich, mit Hilfe der erfindungsgemässen Intracorneallinse Astigmatismus (Hornhautverkrümmung) zu behandeln.

Der Vorteil einer Korrektur dieser Sehschwächen mit der erfindungsgemässen Intracorneallinse besteht darin, dass die Korrektur durch Entfernung der Linse aus der Cornea rückgängig gemacht werden kann. Im Vergleich zu herkömmlichen Korrekturgeräten wie Brillen oder Kontaktlinsen weisen die erfindungsgemässen Intracorneallinsen Vorteile wie Unsichtbarkeit, Vermeidung von Problemen beim Tragen, Vermeidung von Beschlagen von Brillengläsern unter bestimmten Umgebungsbedingungen oder Vermeidung von Irritationen der Cornea beim Tragen von Kontaktlinsen auf.

Die erfindungsgemässe Intracorneallinse weist einen kreisförmigen und kuppelartig gewölbten Grundkörper mit einem Durchmesser im Bereich von 2,4 bis 4 mm, vorzugsweise von 2,7 bis 3,5 mm, insbesondere bevorzugt von 3 mm auf.

Die erfindungsgemässe Intracorneallinse ist vorzugsweise aus einem biokompatiblen Material gefertigt, welches den Durchtritt von Nährflüssigkeiten und anderen Augeninhaltsstoffen sowie eine ausreichende Gasdiffusion (vor allem von Sauerstoff) ermöglicht. Derartige biokompatible permeable Materialien sind dem Fachmann bekannt. Beispielhaft seien Silikone, Hydrogele wie Perfilcon-A®, Urethane oder Acrylate wie Polymethacrylate genannt. Bei der erfindungsgemäss bevorzugten Ausführungsform einer Intracorneallinse mit zentraler Öffnung wird der Durchtritt von Flüssigkeiten und Gasen durch die Linse durch die zentrale Öffnung zusätzlich erleichtert.

Ein wesentlicher Aspekt der erfindungsgemässen Intracorneallinse sind die Krümmungsradien der konvexen vorderen Oberfläche und der konkaven hinteren Oberfläche. Gemäss der vorliegenden Erfindung beziehen sich sämtliche Radiusangaben auf die Linse im hydrierten Zustand, wie er im implantierten Zustand der Linse in der Hornhauttasche vorliegt.

Wie vorstehend ausgeführt weist die konvexe vordere Oberfläche der Linse einen einzigen gleichmässigen Krümmungsradius auf. Im Fall einer erfindungsgemäss bevorzugten Bifokallinse zur Korrektur von Presbyopie liegt der Krümmungsradius der konvexen vorderen Oberfläche vorzugsweise in einem Bereich von 7.5 bis 8 mm. Je nach Anwendungsbereich kann aber ein anderer Krümmungsradius gewählt werden, um die von der erfindungsgemässen Linse an sich hervorgerufene optische Korrektur durch eine Veränderung der Krümmung der äusseren Oberfläche der Cornea zu unterstützen.

Die konkave hintere Oberfläche der erfindungsgemässen Linse weist wie vorstehend ausgeführt einen Krümmungsradius Rcci auf, welcher den mittleren Radius der Cornea geringfügig überschreitet. Der mittlere Radius der Cornea variiert etwas von Individuum zu Individuum und liegt in der Regel in einem Bereich von 7,4 mm bis 8,1 mm, typischerweise bei etwa 7,8 mm. Der Krümmungsradius Rcci der konkaven hinteren Oberfläche der erfindungsgemässen Linse überschreitet den mittleren Radius der Cornea erfindungsgemäss um 0,1 mm bis 2 mm, vorzugsweise 0,2 bis 1,5 mm, insbesondere bevorzugt 0,5 bis 1 mm.

Zur Korrektur bestimmter Sehschwächen ist es erforderlich, die erfindungsgemässe Linse mit mehreren verschiedenen Zonen unterschiedlicher optischer Stärke auszustatten. Diese Zonen werden erfindungsgemäss bevorzugt dadurch realisiert, dass der Krümmungsradius der der hinteren Oberfläche der Linse variiert wird, so dass die Zonen unterschiedliche Dicke und unterschiedliche rückseitige Oberflächenradien aufweisen. Wie vorstehend bereits ausgeführt werden die unterschiedlichen Krümmungsradien bei der erfindungsgemässen Linse auf der konkaven hinteren Oberfläche realisiert. Durch eine Veränderung des Krümmungsradius der Linsenoberfläche wird eine Veränderung der Krümmung der Cornea hervorgerufen, was das Sehvermögen der behandelten Person beeinflusst. Eine Veränderung der Krümmung der äusseren Oberfläche der Cornea hat hierbei einen deutlich höheren Einfluss auf das Sehvermögen, da der Brechungsindex der Cornea sich erheblich vom Brechungsindex der Luft unterscheidet. Hingegen ist der Unterschied der Brechungsindices der Cornea und der Flüssigkeit im Innern des Auges deutlich kleiner. Eine Veränderung der Krümmung der inneren Oberfläche der Cornea hat somit einen etwa um den Faktor 5 geringeren Effekt auf das Sehvermögen als eine entsprechende Veränderung der Krümmung der äusseren Oberfläche der Cornea. Durch Beibehaltung eines gleichmässigen Krümmungsradius der vorderen konvexe Linsenoberfläche und Modifikation des Krümmungsradius der hinteren konkaven Linsenoberfläche kann eine präzisere Einstellung des gewünschten Sehvermögens erreicht werden.

Da die erfindungsgemässe Linse mit mehreren verschiedenen Zonen mit unterschiedlichen Krümmungsradien bereitgestellt wird, ist es wichtig, dass die Linse sich an die Cornea anschmiegt, wie vorstehend beschrieben. Die verschiedenen Krümmungsradien müssen so gewählt sein, dass dieser wesentliche erfindungsgemässe Aspekt erreicht wird. Dies wird dies dadurch erreicht, dass sämtliche verschiedenen Zonen-Krümmungsradien derart gewählt sind, dass jeder dieser Krümmungsradien um 0,1 mm bis 2 mm, vorzugsweise 0,2 bis 1,5 mm, insbesondere bevorzugt 0,5 bis 1 mm grösser ist als der mittlere Radius der Cornea. Es kann aber für bestimmte Anwendungen der erfindungsgemässen Linse ausreichen, dass nur der Krümmungsradius Rcci eines inneren Bereichs der hinteren konkaven Oberfläche diese Anforderung erfüllt, während der Krümmungsradius Rcco von äusseren (am Linsenrand liegenden) Bereichen der hinteren konkaven Oberfläche grösser gewählt werden kann. Erfindungsgemäss bevorzugt liegt der Krümmungsradius Rcco der konkaven hinteren Oberfläche der erfindungsgemässen Linse in einem Bereich von 8.0 mm bis 11.5 mm, vorzugsweise von 8.0 mm bis 10 mm, besonders bevorzugt von 8.0 mm bis 9 mm.

Erfindungsgemäss bevorzugt erfolgt die Veränderung des Krümmungsradius von einer Zone zur nächsten Zone nicht abrupt, sondern über einen Bereich definierter Grösse. Erfindungsgemäss bevorzugt erfolgt der Übergang von einem Krümmungsradius zum nächsten Krümmungsradius innerhalb eines Abschnitts der konkaven hinteren Linsenoberfläche, welcher ein Kugelsegment von 0,1 mm bis 0,5 mm, vorzugsweise von 0,2 bis 0,3 mm darstellt.

Erfindungsgemäss bevorzugt sind die konvexe vordere Linsenoberfläche und die konkave hintere Linsenoberfläche über einen Zwischenabschnitt verbunden, welcher sich kreisförmig über den äusseren Linsenrand erstreckt. Mit Hilfe dieses Zwischenabschnitts werden eine optimale Beibehaltung der Positionierung der Linse innerhalb der Cornea erreicht und etwaige sekundäre optische Effekte an der Linsenkante minimiert beziehungsweise vermieden.

Der erfindungsgemässe Zwischenabschnitt zeichnet sich dadurch aus, dass der Krümmungsradius der vorderen konvexen Oberfläche in einer Entfernung von 0,005 mm bis 0,01 mm, vorzugsweise 0,06 mm bis 0,008 mm von der äusseren Linsenkante abrupt in eine Stufe übergeht, welche zur optischen Achse der Linse um einen Winkel von 15° bis 45°, vorzugsweise von 20° bis 40°, besonders bevorzugt von 30° geneigt ist. Die Stufe hat eine Kantenlänge von vorzugsweise 0,01 mm bis 0,015 mm, besonders bevorzugt 0,01 mm bis 0,013 mm.

Auf der hinteren Oberfläche der Linse ist vorzugsweise kein Zwischenabschnitt vorgesehen. Der Krümmungsradius der äusseren Zone der hinteren Oberfläche wird vorzugsweise bis zur Linsenkante beibehalten.

Die erfindungsgemässe Intracorneallinse weist aufgrund der unterschiedlichen Krümmungsradien der vorderen konvexen Oberfläche und der hinteren konkaven Oberfläche eine gewölbeartige Form auf. Vorzugsweise ist die Form der erfindungsgemässen Linse derart ausgebildet, dass der Mittelpunkt der konvexen vorderen Oberfläche der Linse von einer gedachten geraden Verbindungslinie der Linsenkanten einen Abstand von 0,1 bis 0,2 mm, vorzugsweise 0,13 bis 0,17 mm aufweist.

Gemäss einer bevorzugten Ausführungsform der vorliegenden Erfindung weist die Intracorneallinse eine zentrale Öffnung auf, wie sie in der WO 2009/075685 beschrieben ist.

Die zentrale Öffnung dient zur optimalen Positionierung der erfindungsgemässen Linse in der Hornhauttasche und erleichtert gleichzeitig den Durchtritt von Nährflüssigkeiten und Gasen wie Sauerstoff durch die Linse. Die zentrale Öffnung ist konzentrisch mit der optischen Achse der Linse und erstreckt sich durch die gesamte Linse (d.h. es handelt sich um ein Loch und nicht nur um eine Vertiefung). Die Dimension der zentralen Öffnung ist derart gewählt, dass die Öffnung für den Chirurgen beim Einsetzen der Linse in die Hornhauttasche noch sichtbar ist. Gleichzeitig darf die Öffnung aber nicht so gross sein, dass sie von der behandelten Person bemerkt wird. Es darf also nicht zu irgendwelchen durch die zentrale Öffnung verursachten optischen Effekten kommen. Aus diesem Grund hat die zentrale Öffnung erfindungsgemäss im Fall einer Linse mit einem Durchmesser von 3,0 mm bevorzugt einen Durchmesser von 0,12 mm bis 0,2 mm, vorzugsweise von 0,13 bis 0,17 mm und insbesondere bevorzugt von 0,15 mm. Kleinere Öffnungen können die vorstehend beschriebenen Anforderungen nicht erfüllen, während grössere Öffnungen zu optischen Effekten führen, welche von der behandelten Person wahrgenommen werden, was erfindungsgemäss nicht erwünscht ist. Allgemein darf die zentrale Öffnung der erfindungsgemässen Linse nur einen Anteil der Fläche der vorderen Oberfläche von weniger als 1%, vorzugsweise weniger als 0,5% einnehmen.

Gemäss der vorliegenden Erfindung ist die zentrale Öffnung typischerweise zylindrisch geformt. Aufgrund der Linsendicke an der Position der zentralen Öffnung weist die Öffnung eine Länge von 0,01 bis 0,1 mm, vorzugsweise von 0,015 mm bis 0,05 mm und insbesondere bevorzugt von 0,02 bis 0,03 mm durch die Linse auf. Es können aber auch andere Lochformen gewählt werden, wie sie in der WO 2009/075685 beschrieben und hiermit in Bezug genommen sind. Beispielhaft seien kegelförmige Öffnungen oder Öffnungen mit Abschnitten verschiedenen Durchmesser genannt.

Im Zentrum der Linse (d.h. an ihrer optischen Achse) weist die erfindungsgemässe Linse vorzugsweise eine Dicke von 0,01 bis 0,1 mm, vorzugsweise von 0,015 mm bis 0,05 mm und insbesondere bevorzugt von 0,02 bis 0,03 mm auf.

Wie vorstehend beschrieben kann die erfindungemässe Linse verschiedene Zonen mit unterschiedlicher optischer Kraft aufweisen. Die Zahl der verschiedenen optischen Zonen ist hierbei vom gewünschten Korrektureffekt abhängig, welcher durch die erfindungsgemässe Linse erzielt werden soll.

Gemäss einer bevorzugten erfindungsgemässen Ausführungsform weist die Intracorneallinse zwei optische Zonen auf. Ein erster innerer Bereich erstreckt sich hierbei kreisförmig um das Zentrum der Linse und weist keine optische Kraft auf (nichtoptischer Bereich). Es kann aber für gewisse Anwendungen durchaus erwünscht sein, dass der innere Bereich eine geringe optische Kraft von beispielsweise weniger als 1 Dioptrien, vorzugsweise 0,1 bis 0,8 Dioptrien, besonders bevorzugt 0,1 bis 0,6 Dioptrien aufweist. Für andere Anwendungen kann sogar eine optische Kraft von deutlich über 1 Dioptrie gewählt werden. Erfindungsgemäss bevorzugt weist der innere Bereich einen Durchmesser von 1.4 mm bis 2,4 mm (in Abhängigkeit vom Durchmesser der Linse) auf. Bei einem Linsendurchmesser von 3 mm sollte der innere Bereich vorzugsweise einen Durchmesser von 1,5 bis 2,0 mm, besonders bevorzugt von 1,8 mm aufweisen.

Um den inneren optischen Bereich ist ein kreisförmiger zweiter optischer Bereich angeordnet. Der zweite, äussere Bereich weist eine optische Kraft auf, welche abhängig von der gewünschten Sehkorrektur gewählt ist, welche durch die erfindungsgemässe Linse erzielt werden soll. In der Regel ist die optische Kraft des zweiten äusseren Bereichs grösser als 1 Dioptrien.

Die vorstehend angegebenen Dioptrien-Werte beziehen sich auf die optische Kraft, welche von der Linse im implantierten Zustand, d.h. im hydrierten Zustand, in der Cornea bereitgestellt wird. Gemäss einer weiteren Ausführungsform der vorliegenden Erfindung kann die Intracorneallinse auch als diffraktive Linse ausgebildet sein. Diffraktive Linsen sind aus dem Stand der Technik bekannt. Beispielhaft sei auf die WO 2009/075685 oder die EP-1 001 720 B1 verwiesen. Vorzugsweise ist eine diffraktive Linse derart ausgestaltet, dass sie mehrere kreisförmige optische Zonen aufweist, die über Stufen ineinander übergehen. Dies ist beispielsweise in der WO 2009/075685, Figuren 12 und 13 gezeigt.

Gemäss einer weiteren Ausführungsform der vorliegenden Erfindung kann die Intracorneallinse auch derart ausgestaltet sein, dass sie hinsichtlich ihrer optischen Kraft einen Gradienten aufweist. Dies kann beispielsweise dadurch erreicht werden, dass die Linse aus verschiedenen Materialien mit unterschiedlichen Brechungsindices aufgebaut ist, welche graduell ineinander übergehen. Alternativ kann der Brechungsindex eines Materials auch durch geeignete Massnahmen wie beispielsweise Bestrahlung mit energiereicher elektromagnetischer Strahlung graduell verändert werden.

Aufgrund der unterschiedlichen Ausgestaltung der vorderen Oberfläche und der hinteren Oberfläche der erfindungsgemässen Intracorneallinse ist es ausserordentlich wichtig, dass die erfindungsgemässe Intracorneallinse korrekt in die dafür vorgesehene Tasche in der Cornea eingeführt wird, d.h. mit der vorderen Oberfläche zur Aussenseite des Auges und der hinteren Oberfläche der Linse zum Augeninnern hin.

Das korrekte Einbringen der erfindungsgemässen Linse in eine dafür geschaffene Tasche in der Cornea kann erfindungsgemäss besonders bevorzugt mit einem Applikator durchgeführt werden, wie er in der WO 2011/069907 A1 beschrieben ist.

In der WO 2011/069907 A1 ist ein Applikator beschrieben, der ein Griffstück und eine Pre-Load-Einheit umfasst. Griffstück und Pre-Load-Einheit können miteinander verbunden werden, vorzugsweise mit Hilfe eines verdrehsicheren Bajonett-Verschlusses. Die Pre-Load-Einheit kann vorgängig mit einer Linse wie der erfindungsgemässen Linse bestückt und in einem Aufbewahrungsbehälter steril gelagert werden. Zum Einsetzen der Linse entnimmt der Arzt die Pre-Load-Einheit aus der Aufbewahrungseinheit und verbindet die Pre-Load-Einheit mit dem Griffstück. Anschliessend kann die Linse auf die in der WO 2011/069907 A1 beschriebene Weise in die Hornhauttasche eingeführt werden.

Die vorliegende Erfindung betrifft somit auch ein Kit, umfassend eine Aufbewahrungseinheit und eine Pre-Load-Einheit im Innern der Aufbewahrungseinheit, wobei die Aufbewahrungseinheit aus einem wasserdichten Material besteht und mit einem Stopfen wasserdicht verschliessbar ist und die Pre-Load-Einheit mit einer erfindungsgemässen Intracorneallinse bestückt ist.

Die Pre-Load-Einheit ist vorzugsweise für den Einmalgebrauch konzipiert. Die Pre-Load-Einheit wird mit der erfindungsgemässen Intracorneallinse bestückt und in einer sterilen Verpackung gelagert, aus welcher sie erst unmittelbar vor dem Einsetzen der Linse in das Auge entnommen und mit dem Griffstück verbunden wird.

Um die Pre-Load-Einheit über eine längere Zeit steril lagern zu können, wird sie in einer Aufbewahrungseinheit verpackt, welche die Pre-Load-Einheit vor Umwelteinflüssen schützt. Zu diesem Zweck ist die Aufbewahrungseinheit im Innern mit einer Lagerflüssigkeit gefüllt, welche mindestens die in der Kammer der Pre-Load-Einheit befindliche Linse ständig bedeckt. Es kann sich dabei um Wasser handeln; bevorzugt ist aber physiologische Kochsalzlösung (NaCl) als Lagerflüssigkeit.

Die Aufbewahrungseinheit ist aus einem Material gefertigt, welches wasserdicht ist und somit auch Wasserdampf wenn überhaupt nur in sehr geringem Mass passieren lässt. Dies ist wichtig, um einen ausreichenden Pegel an Lagerflüssigkeit innerhalb der Aufbewahrungseinheit während der gesamten Lagerungszeit zu gewährleisten. Bei nicht wasserdichtem Material würde die Lagerflüssigkeit über einen gewissen Zeitraum verdampfen, und die Linse in der Kammer der Pre-Load-Einheit wäre nicht mehr steril gelagert. Wasserdichte Materialien sind dem Fachmann bekannt. Als Beispiel seien Glas oder ein wasserdichter Kunststoff genannt. Nach Einbringen der Pre-Load-Einheit wird die Aufbewahrungseinheit wasserdicht mit einem geeigneten Stopfen verschlossen. Dieser Stopfen ist vorzugsweise aus einem wasserdichten Kunststoff gefertigt.

Die Oberseite der Pre-Load-Einheit ist deutlich sichtbar gekennzeichnet, beispielsweise mit einer entsprechenden Aufschrift wie "TOP" oder "OBEN". Da die Pre-Load-Einheit mit der erfindungsgemässen Linse derart bestückt ist, dass die konvexe vordere Oberfläche der Linse in Richtung der gekennzeichneten Oberseite der Pre-Load-Einheit weist, stellt das korrekte Einsetzen der Linse in die Tasche der Cornea für den Chirurgen kein Problem da. Er muss lediglich die Pre-Load-Einheit korrekt auf das Griffstück aufsetzen, d.h. mit der gekennzeichneten Oberseite der Pre-Load-Einheit nach oben ausgerichtet.

Die Pre-Load-Einheit ist in der WO 2011/069907 A1 ausführlich beschrieben. Die Pre-Load-Einheit zur Einführung von Linsen in das Auge eines Menschen oder Tieres umfasst
i) ein Gehäuse mit Mitteln zur, vorzugsweise verdrehsicheren, Befestigung der Einheit an einem Griffstück,
ii) ein Linsenaufnahmeteil, welches an oder in dem Gehäuse angeordnet ist und einen aus dem Gehäuse herausstehenden Abschnitt mit vorzugsweise genau zwei getrennten blattartigen Einheiten umfasst, welche zumindest an ihren vom Gehäuse entfernten Enden miteinander in lösbarem Kontakt stehen und dort eine Kammer zur Aufbewahrung einer optischen Linse bilden,
iii) einen Schieber, welcher beweglich im Innern des Gehäuses angeordnet ist und zwischen den blattartigen Einheiten der Linsenaufnahmeeinheit bewegt werden kann.

Gemäss der vorliegenden Erfindung soll unter einer blattartigen Einheit ein Bauteil verstanden werden, welches analog zu einem Blatt sehr dünn ist und damit eine entsprechende Flexibilität aufweist. Gemäss der vorliegenden Erfindung sind die blattartigen Einheiten typischerweise zwischen 0,1 und 0,3 mm dick und typischerweise 20-40, vorzugsweise 20 bis 30 mm lang. Als Material für die blattartigen Einheiten kommt ein physiologisch verträglicher Kunststoff oder ein physiologisch verträgliches Metall in Frage.

Die zwei blattartigen Einheiten weisen vorzugsweise jeweils genau ein Loch auf, welche so angeordnet sind, dass die Löcher direkt übereinander liegen und eine Öffnung durch das Zentrum der Kammer zur Aufbewahrung einer optischen Linse bilden. Auf diese Weise ist es besonders gut möglich, eine in der Kammer befindliche Linse auf der Sehachse des Auges zu positionieren. Die das Einsetzen der Linse vornehmende Person sieht die Linse durch die Öffnung und kann sie exakt ausrichten. Diese Löcher können typischerweise einen Durchmesser von 0,3 bis 0,6 mm aufweisen. Diese Ausführungsform ist besonders für Intracorneallinsen mit einem zentralen Loch vorteilhaft, wie sie vorstehend beschrieben sind.

Die blattartigen Einheiten des Linsenaufnahmeteils bilden an ihren vom Gehäuse entfernten Enden eine Kammer zur Aufbewahrung der erfindungsgemässen Linse. Die blattartigen Einheiten befinden sich im Bereich der Kammer in losem Kontakt miteinander, d.h. sie können durch Kraftanwendung voneinander weg bewegt werden, um die Kammer zum Einführen und Entnehmen der erfindungsgemässen Linse zu öffnen. Die blattartigen Einheiten werden mittels eines Schiebers auseinander gespreizt. Die Kammer ist zumindest teilweise durch wellenförmige Abschnitte begrenzt, welche auf beiden blattartigen Einheiten vorhanden und so angeordnet sind, dass die Abschnitte der einen blattartigen Einheit mit den entsprechenden Abschnitten der anderen blattartigen Einheit verzahnt sind. Im Zustand des Kontakts der beiden blattartigen Einheiten ist ein Verrutschen der Linse aus der Kammer nicht möglich.

Mit dem in der WO 2011/069907 A1 beschriebenen Applikator wird die Linse nicht in eine Hornhauttasche gestossen, sondern vielmehr wird der Applikator in der Hornhauttasche exakt positioniert, und anschliessend werden die blattartigen Teile unter Orterhalt der Linse um einen genau definierten Betrag zurückgezogen. Hierfür ist der Abschnitt der Pre-Load-Einheit des Applikators, in welchem die Kammer zur Aufbewahrung der erfindungsgemässen Linse ausgebildet ist, derart geformt, dass er in eine entsprechende Hornhauttasche eingeführt werden kann. Bevorzugt entspricht die Form des diese Kammer bildenden Bereichs des Applikators im wesentlichen der Form der zu platzierenden Linse beziehungsweise ist in seiner Breite sogar geringfügig kleiner als die entsprechende Linse. Dies ist zum Beispiel dadurch möglich, dass die Kammer keine runde Form hat und nicht überall die vorstehend beschriebenen Begrenzungen aufweist, d.h. zwischen den Begrenzungen ist die Kammer seitlich offen. Die in der Kammer befindliche Linse kann dann an einigen Stellen aus der Kammer herausragen.

Um die Pre-Load-Einheit mit der erfindungsgemässen Linse zu beschicken, werden die blattartigen Einheiten voneinander weg gespreizt, um einen Zugang zur vorstehend beschriebenen Kammer zu ermöglichen. Gemäss einer Ausführungsform der vorliegenden Erfindung kann man hierfür ein geeignetes Werkzeug, beispielsweise ein Messer, in einen seitlichen Zwischenraum zwischen den blattartigen Einheiten einführen. Anschliessend wird die Linse, vorzugsweise unter einem Mikroskop, in die zugängliche Kammer platziert. Danach wird das Werkzeug aus dem seitlichen Zwischenraum zwischen den blattartigen Einheiten entfernt, wodurch die blattartigen Einheiten an ihren Enden wieder in Kontakt zueinander kommen und die Kammer geschlossen wird. Für den Beladungsvorgang kann man beispielsweise eine Beladungsstation bereitstellen, in welcher die Pre-Load-Einheit fixiert werden kann. Die Beladungsstation weist ein drehbares Werkzeug (z.B. ein Messer) auf, welches in den seitlichen Zwischenraum zwischen den blattartigen Einheiten hineingedreht und aus diesem wieder herausgedreht werden kann.

Nach Entnahme aus der Aufbewahrungseinheit wird die Pre-Load-Einheit auf ein Griffstück aufgesetzt, welches ebenfalls in der WO 2011/069907 A1 ausführlich beschrieben ist. Typischerweise handelt es sich hierbei um ein längliches Rohr mit einer Form, welche ein einfaches Halten des Griffstücks in der Hand gewährleistet. Das Griffstück ist an einem Ende derart ausgebildet, dass die Pre-Load-Einheit und das Griffstück derart miteinander verbunden werden können, dass die Pre-Load-Einheit innerhalb des Griffstücks bewegt werden kann. Hierzu weist das Griffstück einen Durchmesser auf, welcher den Durchmesser des in das Griffstück einzuführenden Teils der Pre-Load-Einheit übersteigt. Zumindest an dem mit der Pre-Load-Einheit zu verbindenden Ende ist das Griffstück innen hohl. Vorzugsweise handelt es sich aber um ein vollständig hohles Rohr. Innerhalb der Pre-Load-Einheit und des Griffstücks ist ein Schieber beweglich angeordnet. Dieser Schieber muss zwischen den blattartigen Einheiten so weit nach vorne bewegt werden können, dass er in Kontakt mit der in der Kammer befindlichen Linse kommt und diese fixieren kann. Vorzugsweise verjüngt sich der Schieber zu dem zwischen den blattartigen Einheiten zu bewegenden Ende hin. Zur besseren Fixierung der Linse ist das vordere Ende des Schiebers entsprechend komplementär zur Linsenform ausgebildet.

Schieber und Pre-Load-Einheit werden mit Hilfe von Bedienelementen bewegt, die am Griffstück angeordnet sind. Dies ist in der WO 2011/069907 A1 ausführlich beschrieben.

Das Griffstück des erfindungsgemässen Applikators ist vorzugsweise für eine mehrfache Anwendung vorgesehen. Zu diesem Zweck ist das Griffstück gemäss einer bevorzugten Ausführungsform der vorliegenden Erfindung vollständig demontierbar, so dass sämtliche Bauteile vollständig gereinigt und sterilisiert werden können. Gemäss einer alternativen Ausführungsform der vorliegenden Erfindung ist es aber auch möglich, das Griffstück ebenfalls für den Einmalgebrauch vorzusehen. Gemäss dieser Ausführungsform wird das Griffstück vorzugsweise separat ebenfalls in einer Aufbewahrungseinheit analog der vorstehend beschriebenen Einheit bis zur Verwendung aufbewahrt. Es ist gemäss dieser Ausführungsform aber auch denkbar, dass die Pre-Load-Einheit und das Griffstück bereits als Einheit (entweder wie vorstehend beschrieben trennbar oder alternativ auch fest miteinander verbunden) in einer Aufbewahrungseinheit analog der vorstehend beschriebenen Einheit bis zur Verwendung aufbewahrt werden. Der Applikator gemäss dieser alternativen Ausführungsform kann daher anders aufgebaut sein, indem die vorstehenden Teile zur Verbindung von Griffstück und Pre-Load-Einheit durch eine feste Verbindung dieser Bauteile ersetzt sein können.

Mit Hilfe des vorstehend beschriebenen Applikators der WO 2011/069907 A1 kann die erfindungsgemässe Linse auf einfache Weise in eine Tasche in der Cornea eingeführt werden. Das Einsetzverfahren umfasst die Schritte:
a) Positionieren des vorstehend beschriebenen Applikators an der gewünschten Stelle des Auges in der korrekten Ausrichtung, vorzugsweise in einer Tasche in der menschlichen Cornea, so dass das Zentrum der im Applikator enthaltenen optischen Linse auf der Sehachse des Auges liegt;
b) Vorschieben des Schiebers mittels eines ersten Bedienelements am Griffstück, bis der Schieber in Kontakt mit der Linse kommt ohne diese zu bewegen, wobei gleichzeitig die blattartigen Einheiten des Linsenaufnahmeteils voneinander weg gespreizt werden;
c) Zurückziehen der restlichen Pre-Load-Einheit um einen definierten Betrag mittels eines zweiten Bedienelements am Griffstück unter gleichzeitiger Fixierung des Schiebers, wodurch die Linse aus dem Applikator freigesetzt wird.

Die vorzugsweise steril gelagerte Pre-Load-Einheit wird aus dem Aufbewahrungsbehälter entnommen und mit dem Griffstück vorzugsweise verdrehsicher verbunden. Anschliessend wird der so erhaltene Applikator mit der bestückten erfindungsgemässen Linse an der gewünschten Stelle des Auges positioniert. Dazu werden die Enden der blattartigen Einheiten der Pre-Load-Einheit, welche die Kammer mit der darin befindlichen erfindungsgemässen Linse bilden, in einer zuvor erzeugten Tasche in der Hornhaut eines Auges eingeführt. Gemäss einer besonders bevorzugten Ausführungsform der vorliegenden Erfindung weist die Pre-Load-Einheit eine durch die Kammer mit der Linse gehende Öffnung auf. Dies ermöglicht der das Verfahren durchführenden Person, die Linse exakt auf der Sehachse des Auges auszurichten, während sich die Linse noch in der Kammer des Applikators befindet. Wie vorstehend ausgeführt ist es ausserordentlich wichtig, dass der Applikator und damit die im Applikator befindliche Linse korrekt ausgerichtet in die Hornhauttasche eingeführt werden. Dies wird durch eine deutliche Kennzeichnung der Oberseite der Pre-Load-Einheit mit darin korrekt platzierter Linse erreicht. Der Chirurg muss darauf achten, dass der korrekt mit der Linse bestückte Applikator (d.h. die vordere Oberfläche der Linse ist zu der deutlich gekennzeichneten Oberseite der Pre-Load-Einheit ausgerichtet) richtig in die die Hornhauttasche eingeführt wird, d.h. mit der gekennzeichneten Oberseite der Pre-Load-Einheit nach oben weisend (vom Augeninnern weg).

Anschliessend wird die Linse innerhalb des Applikators fixiert, indem das erste Bedienelement am Griffstück vollständig nach vorne geschoben wird, wodurch der Schieber der Pre-Load-Einheit maximal nach vorne bewegt wird, so dass er in Kontakt mit der in der Kammer befindlichen Linse kommt. Dies wird dadurch möglich, weil durch das Vorwärtsbewegen des Schiebers die blattartigen Einheiten voneinander weg gespreizt werden und somit das Innere der Kammer zugänglich wird. Im nächsten Schritt wird nun die restliche Pre-Load-Einheit ausgenommen vom Schieber mit Hilfe des zweiten Bedienelements vom Auge weg um einen definierten Betrag zurückgezogen, indem die restliche Pre-Load-Einheit in das Griffstück hinein gezogen wird. Während dieses Schritts bleibt der Schieber fixiert und hält somit die Linse exakt an der zuvor ausgerichteten Position. Durch das Zurückziehen der restlichen Pre-Load-Einheit wird die Linse aus dem Applikator exakt in der zuvor ausgerichteten Position freigesetzt. Die Linse ist somit bereits wie gewünscht im Auge platziert. Eines weiteren Ausrichtungsschrittes bedarf es nicht mehr. Anschliessend wird der Applikator vom Auge entfernt und in seine Einzelteile zerlegt. Vorzugsweise wird die für den Einmalgebrauch vorgesehene Pre-Load-Einheit nun entsorgt, während das Griffstück zerlegt und gereinigt wird, so dass es für den nächsten Einsatz mit einer neuen Pre-Load-Einheit zur Verfügung steht.

Verfahren zur Erzeugung von Taschen in der Hornhaut zur Implantierung von Intracorneallinsen sind bekannt. Beispielhaft sei auf spezifische Schneidinstrumente (Mikrokeratome), wie sie in der EP-1 778 141 A1 beschrieben sind, sowie auf Lasertechniken verwiesen. Die Erzeugung von Taschen in der Cornea mit Hilfe von Lasern (wie gepulsten Lasern) sind beispielsweise in der US 2003/0014042 A1 oder in der WO2008/072092 A1 beschrieben. Insbesondere mit Hilfe von Lasern können Taschen in der Cornea mit sehr präziser Schnitttiefe erzeugt werden, wodurch eine sehr exakte Positionierung der erfindungsgemässen Linsen und damit einhergehend eine sehr präzise Korrektur der entsprechenden Sehschwäche erreicht werden kann.

Die vorliegende Erfindung wird nachstehend anhand von nicht einschränkenden Zeichnungen und Beispielen näher erläutert. Es zeigen:
- Fig. 1: eine Vorderansicht einer Ausführungsform einer erfindungsgemässen Intracorneallinse
- Fig. 2: eine nicht masstabsgetreue Seitenansicht der erfindungsgemässen Intracorneallinse gemäss Fig. 1
- Fig. 3: eine vergrösserte Darstellung des Abschnitts X der erfindungsgemässen Intracorneallinse gemäss Fig. 2
- Fig. 4: eine Ausführungsform des Applikators gemäss der WO 2011/069907 A1 zur Einführung der erfindungsgemässen Linse in eine Hornhauttasche
- Fig. 5: eine Ausführungsform des Kits gemäss der WO 2011/069907 A1 zur Aufbewahrung der mit der erfindungsgemässen Linse bestückten Pre-Load-Einheit
- Fig. 6: eine Detailansicht der Funktionsweise des Applikators gemäss der WO 2011/069907 A1

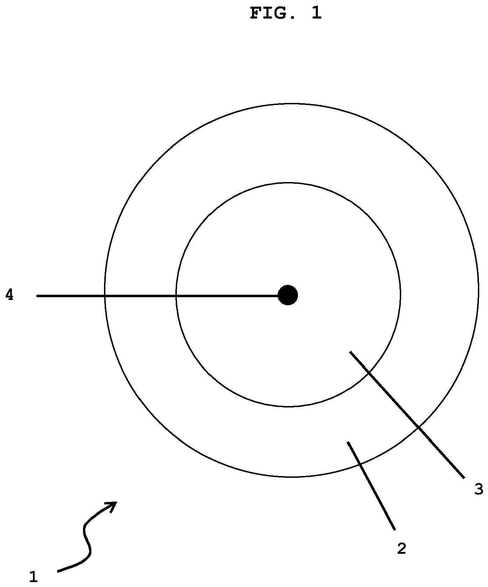

In Fig. 1 ist einer Vorderansicht einer Ausführungsform einer erfindungsgemässen Intracorneallinse 1 gezeigt. Die Linse 1 weist in dieser Ausführungsform einen Durchmesser von 3,0 mm auf. Die Linse weist einen äusseren optischen Bereich 2 auf, der in dieser Ausführungsform eine optische Kraft von 1,25 Dioptrien (in der Cornea) besitzt. Weiterhin weist die Linse 1 einen inneren Bereich 3 auf, der in dieser Ausführungsform einen Durchmesser von 1,8 mm aufweist und keine optische Kraft oder in anderen Anwendungen eine optische Kraft von maximal 0,5 Dioptrien besitzt. Die Linse 1 weist zudem eine zentrale Öffnung 4 auf. Diese ist konzentrisch zur optischen Achse der Linse 1 angeordnet und weist in dieser Ausführungsform einen Durchmesser von 0,15 mm auf.

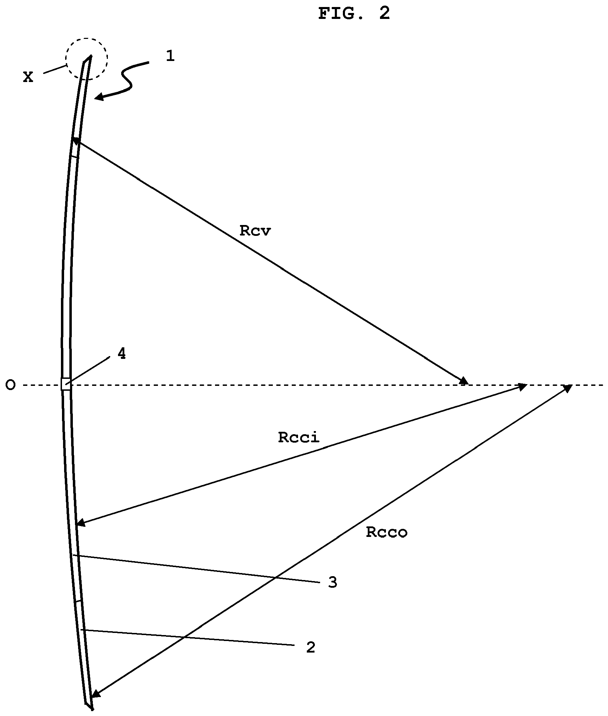

In Fig. 2 ist einer Seitenansicht einer Ausführungsform einer erfindungsgemässen Intracorneallinse 1 gezeigt. Die Krümmungsradien sind in der Fig. 2 nicht massstabsgetreu gezeigt. Die Linse 1 weist eine konvexe vordere Oberfläche mit einem durchgehenden gleichmässigen Krümmungsradius Rcv auf. Die verschiedenen Zonen 2 und 3 sind durch unterschiedliche Krümmungsradien Rcco und Rcci auf der konkaven hinteren Oberfläche der Linse 1 ausgezeichnet. In der Ausführungsform gemäss Fig. 2 weisen die Krümmungsradien folgende Werte auf: Rcv: 7,8 mm; Rcco:8,858 mm; Rcci: 8,192 mm. Der Krümmungsradius Rcco geht innerhalb eines Kreissegments von 0,027 mm auf der hinteren Oberfläche der Linse 1 in den Krümmungsradius Rcci über. Konzentrisch mit der optischen Achse O ist in der Linse 1 eine zentrale Öffnung 4 ausgebildet, welche eine Tiefe von 0,0259 mm aufweist. Die konvexe vordere Oberfläche der Linse 1 ist mit der konkaven hinteren Oberfläche der Linse 1 über einen Zwischenabschnitt verbunden, welcher sich in dem durch den gestrichelten Kreis X gekennzeichneten Bereich am Linsenrand befindet.

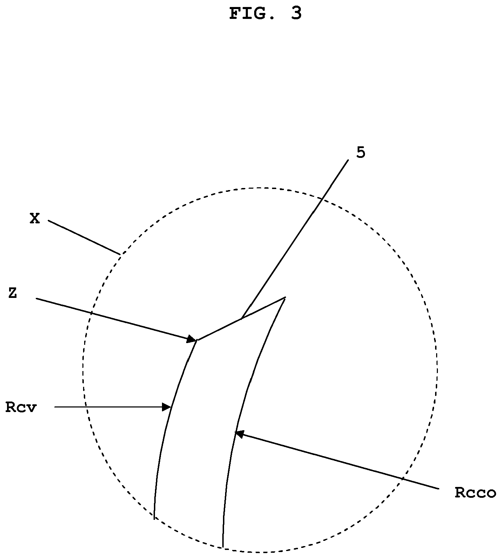

In Fig. 3 ist eine vergrösserte Ansicht des Zwischenabschnitts der erfindungsgemässen Linse 1 aus dem gestrichelten Kreis X der Fig. 2 gezeigt. Die vordere konvexe Oberfläche der Linse 1 weist den Krümmungsradius Rcv bis zu einem Punkt Z auf der vorderen Oberfläche der Linse 1 auf. Dieser Punkt Z befindet sich in der Ausführungsform gemäss Fig. 3 0,0078 mm unterhalb der Kante der Linse 1. Ab dem Punkt Z weist die vordere Oberfläche der Linse 1 eine gerade Form auf. Dieser gerade Abschnitt verbindet den Punkt Z auf der vorderen Oberfläche der Linse 1 mit der Linsenkante, wo die hintere Oberfläche der Linse 1 mit dem Krümmungsradius Rcco ihren Ursprung hat. Der gerade Abschnitt zwischen dem Punkt Z und der Linsenkante ist in der Ausführungsform gemäss Fig. 3 um 30° gegenüber der optischen Achse O der Linse geneigt. Der gerade Abschnitt hat in der Ausführungsform gemäss Fig. 3 eine Länge von 0,012 mm.

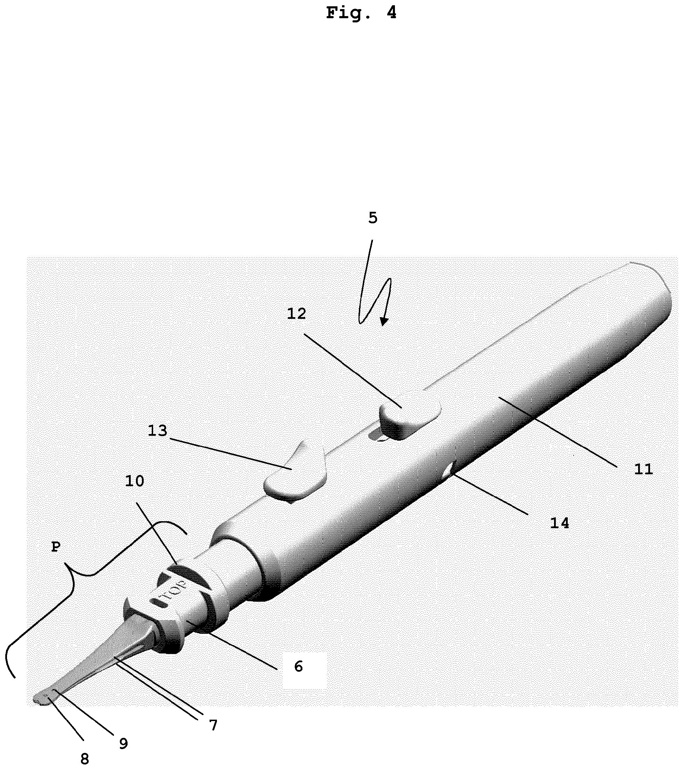

In Fig. 4 ist eine Ausführungsform des Applikators 5 aus der WO 2011/069907 A1 gezeigt. In dem Griffstück 11 ist eine Pre-Load-Einheit P beweglich angebracht. Die Pre-Load-Einheit P umfasst ein Gehäuse 6, zwei blattartige Einheiten 7 und einen Stopper 10, welcher das Hineinbewegen der Pre-Load-Einheit P in das Griffstück 11 begrenzt. In den blattartigen Einheiten 7 ist eine durchgehende Öffnung 8 durch das Zentrum der (hier nicht erkennbaren) Kammer zur Aufnahme einer Linse vorhanden. Zudem ist ein weiteres Loch 9 bereitgestellt, welches das Beschicken des Applikators 7 mit der erfindungsgemässen Linse vereinfacht. Auf dem Griffstück 11 sind zwei Bedienelemente 12 und 13 angebracht, mit deren Hilfe die Pre-Load-Einheit P sowie ein in Fig. 4 nicht sichtbarer Schieber im Innern der Pre-Load-Einheit P und des Griffstücks 11 bewegt werden können. Aus der seitlichen Öffnung 14 ragt ein Stift heraus, welcher an im Griffstück vorhandenen Einlegeteilen befestigt ist und diese im Griffstück 11 fixiert. Die Oberseite der Pre-Load-Einheit P ist deutlich mit dem Wort "TOP" gekennzeichnet.

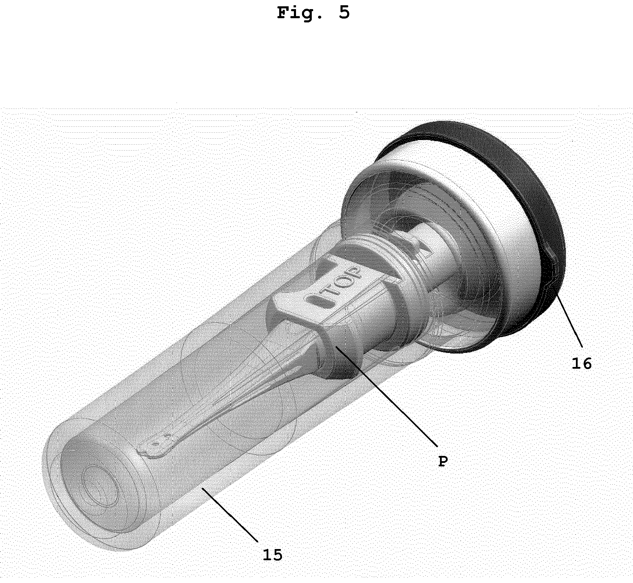

In Fig. 5 ist eine Ausführungsform des Kits gemäss der WO 2011/069907 A1 gezeigt, welches eine Aufbewahrungseinheit 15 umfasst. Im Innern der Aufbewahrungseinheit 15 ist die Pre-Load-Einheit P in einer physiologischen Kochsalzlösung gelagert. Die Aufbewahrungseinheit 15 ist aus Glas. Sie ist mit einem Stopfen 16 wasserdicht verschlossen.

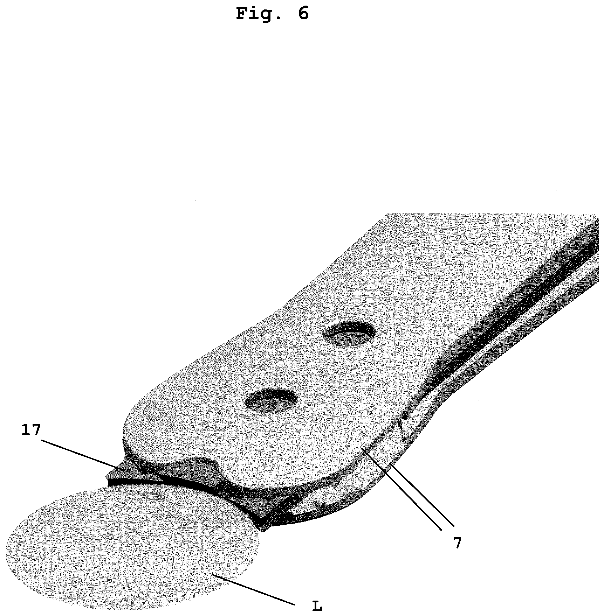

In Fig. 6 ist gezeigt, wie eine Linse L aus der Pre-Load-Einheit gemäss der WO 2011/069907 A1 freigesetzt wird. Der Schieber 17 ist so weit vorgeschoben, dass er in Kontakt zu der Linse L steht. Dadurch sind die blattartigen Einheiten 7 voneinander weg gespreizt. Die blattartigen Einheiten 7 können nun um einen definierten Betrag zurückgezogen werden, während der Schieber 17 die Linse L an dem vorher ausgerichteten Ort hält. Die Linse L ist nun freigesetzt, und der erfindungsgemässe Applikator kann entfernt werden.

Einer Gruppe von 28 Probanden wurde eine erfindungsgemässe Intracorneallinse eingesetzt, wie sie in den Figuren 1 bis 3 beschrieben ist. Die Probanden waren anschliessend in einem Standard-Sehtest in der Lage, aus einer Entfernung von 25 cm etwa 4 Linienpaare pro mm aufgelöst zu erkennen. Aus Vergleichsversuchen mit einer Intracorneallinse entsprechend der WO 2009/075685 ist bekannt, dass mit einer derartigen herkömmlichen Linse im analogen test nur eine Auflösung von etwa 2 Linienpaaren pro mm erreicht werden kann.

## Patentansprüche

1. Intracorneallinse (1), umfassend einen kreisförmigen Grundkörper mit einer konvexen vorderen Oberfläche und einer konkaven hinteren Oberfläche, in dem die konvexe vordere Oberfläche einen einzigen gleichmässigen Krümmungsradius (Rcv) aufweist, und wobei die konkave hintere Oberfläche einen inneren optischen Bereich(3) mit einem Krümmungsradius (Rcci) und einen äusseren optischen Bereich (2) mit einem Krümmunsradius (Rcco), der unterschiedlich zum Krümmungsradius (Rcci) des inneren Bereiches (3) ist, aufweist, und wobei der Krümmungsradius (Rcci) des inneren optischen Bereichs (3) um 0,1 mm bis 2 mm, vorzugsweise 0,2 bis 1,5 mm, insbesondere bevorzugt 0,5 bis 1 mm grösser ist als der mittlere Radius der Cornea, welcher abhängig vom Individuum in einem Bereich von 7,4 mm bis 8,1 mm liegt.

2. Intracorneallinse nach Anspruch 1, wobei die Linse (1) eine zentrale Öffnung (4) aufweist, welche konzentrisch zur optischen Achse ist und einen Anteil der Fläche der vorderen Oberfläche von weniger als 1%, vorzugsweise weniger als 0,5% einnimmt.

3. Intracorneallinse nach einem der vorhergehenden Ansprüche, wobei der kreisförmige Grundkörper einen Durchmesser im Bereich von 2,4 bis 4 mm, vorzugsweise von 2,7 bis 3,5 mm aufweist.

4. Intracorneallinse nach einem der vorhergehenden Ansprüche, wobei die Linse (1) im Bereich ihrer optischen Achse eine Dicke im Bereich von 0,01 bis 0,1 mm, vorzugsweise von 0,015 mm bis 0,05 mm und insbesondere bevorzugt von 0,02 bis 0,03 mm aufweist.

5. Intracorneallinse nach einem der vorhergehenden Ansprüche, wobei die konvexe vordere Oberfläche und die konkave hintere Oberfläche über einen Zwischenabschnitt am äusseren Rand des kreisförmigen Grundkörpers miteinander verbunden sind.

6. Intracorneallinse nach Anspruch 5, wobei der innere optische Bereich (3) keine optische Korrektur bereitstellt.

7. Intracorneallinse nach einem der Ansprüche 1 bis 5, wobei es sich um eine diffractive Linse handelt.

8. Intracorneallinse nach einem der Ansprüche 1 bis 5 oder 7, wobei
(i) der erste Krümmungsradius (Rcci) des inneren optischen Bereiches (3) eine optische Kraft von weniger als 1 Dioptrien aufweist, und/oder
(ii) der zweite Krümmungsradius (Rcco) des äusseren optischen Bereiches eine optische Kraft von grösser als 1 Dioptrien aufweist.

9. Intracorneallinse nach einem der Ansprüche 1 bis 5 oder 7 bis 8, wobei der innere optische Bereich (3) eine optische Kraft von maximal 0.5 Dioptrien aufweist und der äussere optische Bereich eine optische Kraft von 1.25 Dioptrien aufweist und wobei die Linse einen Durchmesser von 3,0 mm und der innere optische Bereich (3) einen Durchmesser von 1,8 mm aufweist.

10. Kit, umfassend eine Aufbewahrungseinheit (15) und eine Pre-Load-Einheit (P) im Innern der Aufbewahrungseinheit (15), wobei die Aufbewahrungseinheit (15) aus einem wasserdichten Material besteht und mit einem Stopfen (16) wasserdicht verschliessbar ist, und die Pre-Load-Einheit (P) mit einer Intracorneallinse germäss einem der Anspruche 1 bis 9 bestückt ist.

11. Kit nach Anspruch 10, wobei der Innenraum der Aufbewahrungseinheit (15) mit physiologischer Kochsalzlösung gefüllt ist.

12. Kit nach Anspruch 10 oder 11, wobei die Pre-Load-Einheit (P) umfasst:
i) ein Gehäuse (6) mit Mitteln zur, vorzugsweise verdrehsicheren, Befestigung der Einheit an einem Griffstück (11),
ii) ein Linsenaufnahmeteil, welches an oder in dem Gehäuse (6) angeordnet ist und einen aus dem Gehäuse (6) herausstehenden Abschnitt mit zwei getrennten blattartigen Einheiten (7) umfasst, welche zumindest an ihren vom Gehäuse (6) entfernten Enden miteinander in lösbarem Kontakt stehen und dort eine Kammer zur Aufbewahrung einer Linse gemäss einem der Ansprüche 1 bis 9 bilden,
iii) einen Schieber (17), welcher beweglich im Innern des Gehäuses (6) angeordnet ist und zwischen den blattartigen Einheiten (7) der Linsenaufnahmeeinheit bewegt werden kann.

13. Kit nach Anspruch 12, wobei die zwei blattartigen Einheiten (7) jeweils ein Loch (8) aufweisen, welche so angeordnet sind, dass die Löcher (8) direkt über- einander liegen und eine Öffnung durch das Zentrum der Kammer zur Aufbewahrung einer Linse gemäss einem der Ansprüche 1 bis 9 bilden.

14. Kit nach einem der Ansprüche 10 bis 13, wobei zusätzlich ein Griffstück (11) zur Verbindung mit der Pre-Load-Einheit (P) bereitgestellt ist.

15. Kit nach Anspruch 14, wobei das Griffstück (11) derart mit der Pre-Load-Einheit (P verbunden werden kann, dass die Pre-Load-Einheit (P) im verbundenen Zustand innerhalb des Griffstücks (11) bewegt werden kann, und dass am Griffstück (11) Bedienelemente(12,13) zur Bewegung des Schiebers (17) und der restlichen Pre-Load-Einheit (P) vorgesehen sind.

## Claims

1. Intracorneal lens (1), comprising a circular main body having a convex front surface and a concave rear surface, in which the convex front surface has a single uniform radius of curvature (Rcv), wherein the concave rear surface has an inner optical region (3) having a radius of curvature (Rcci), and an outer optical region (2) having a radius of curvature (Rcco) which is different from the radius of curvature (Rcci) of the inner region (3), and wherein the radius of curvature (Rcci) of the inner optical region (3) is 0.1 mm to 2 mm, preferably 0.2 to 1.5 mm, particularly preferably 0.5 to 1 mm, greater than the average radius of the cornea, which, depending on the individual, is in a range of from 7.4 mm to 8.1 mm.

2. Intracorneal lens according to claim 1, wherein the lens (1) has a central opening (4) which is concentric to the optical axis and occupies a proportion of the area of the front surface of less than 1%, preferably less than 0.5%.

3. Intracorneal lens according to any of the preceding claims, wherein the circular main body has a diameter in the range of from 2.4 to 4 mm, preferably from 2.7 to 3.5 mm.

4. Intracorneal lens according to any of the preceding claims, wherein the lens (1) has a thickness in the range from 0.01 to 0.1 mm, preferably from 0.015 mm to 0.05 mm and particularly preferably from 0.02 to 0.03 mm in the region of the optical axis thereof.

5. Intracorneal lens according to any of the preceding claims, wherein the convex front surface and the concave rear surface are interconnected via an intermediate portion on the outer edge of the circular main body.

6. Intracorneal lens according to claim 5, wherein the inner optical region (3) does not provide optical correction.

7. Intracorneal lens according to any of claims 1 to 5, wherein said lens is a diffractive lens.

8. Intracorneal lens according to any of claims 1 to 5 or 7, wherein
(i) the first radius of curvature (Rcci) of the inner optical region (3) has an optical power of less than 1 dioptre, and/or
(ii) the second radius of curvature (Rcco) of the outer optical region has an optical power greater than 1 dioptre.

9. Intracorneal lens according to any of claims 1 to 5 or 7 to 8, wherein the inner optical region (3) has an optical power of at most 0.5 dioptres and the outer optical region has an optical power of 1.25 dioptres and wherein the lens has a diameter of 3.0 mm and the inner optical region (3) has a diameter of 1.8 mm.

10. Kit comprising a storage unit (15) and a pre-load unit (P) inside the storage unit (15), wherein the storage unit (15) is made of a waterproof material and can be closed in a watertight manner by means of a stopper (16), and the pre-load unit (P) is equipped with an intracorneal lens according to any of claims 1 to 9.

11. Kit according to claim 10, wherein the interior of the storage unit (15) is filled with physiological saline solution.

12. Kit according to either claim 10 or claim 11, wherein the pre-load unit (P) comprises:
i) a housing (6) having means for fastening the unit to a handle piece (11), preferably in a non-rotatable manner,
ii) a lens receiving part which is arranged on or in the housing (6) and comprises a portion which projects from the housing (6) and has two separate laminar units (7) which are in detachable contact with one another, at least at the ends thereof that are remote from the housing (6), and form a chamber at said ends for storing a lens according to any of claims 1 to 9,
iii) a slide (17) which is movably arranged inside the housing (6) and can be moved between the laminar units (7) of the lens receiving unit.

13. Kit according to claim 12, wherein the two laminar units (7) each have a hole (8), which holes (8) are arranged such that they are directly above one another and form an opening through the centre of the chamber for storing a lens according to any of claims 1 to 9.

14. Kit according to any of claims 10 to 13, wherein a handle piece (11) for connection to the pre-load unit (P) is additionally provided.

15. Kit according to claim 14, wherein the handle piece (11) can be connected to the pre-load unit (P) in such a way that the pre-load unit (P) can be moved inside the handle piece (11) in the connected state, and that operating elements (12, 13) for moving the slide (17) and the remainder of the pre-load unit (P) are provided on the handle piece (11).

## Revendications

1. Lentille intracornéenne (1) comprenant un corps de base circulaire comportant une surface avant convexe et une surface arrière concave, la surface avant convexe présentant un rayon de courbure unique uniforme (Rcv), et la surface arrière concave présentant une zone optique intérieure (3) comportant un rayon de courbure (Rcci) et une zone optique extérieure (2) comportant un rayon de courbure (Rcco) qui est différent du rayon de courbure (Rcci) de la zone intérieure (3), et le rayon de courbure (Rcci) de la zone optique intérieure (3) étant plus grand de 0,1 à 2 mm, de préférence de 0,2 à 1,5 mm, plus préférentiellement de 0,5 à 1 mm plus grand que le rayon moyen de la cornée, qui est compris entre 7,4 et 8,1 mm selon les individus.

2. Lentille intracornéenne selon la revendication 1, dans laquelle la lentille (1) présente une ouverture centrale (4) qui est concentrique à l'axe optique et occupe une proportion de la superficie de la surface avant inférieure à 1 %, de préférence inférieure à 0,5 %.

3. Lentille intracornéenne selon l'une des revendications précédentes, dans laquelle le corps de base circulaire présente un diamètre compris entre 2,4 et 4 mm, de préférence entre 2,7 et 3,5 mm.

4. Lentille intracornéenne selon l'une des revendications précédentes, dans laquelle la lentille (1) présente, dans la zone de son axe optique, une épaisseur comprise entre 0,01 et 0,1 mm, de préférence entre 0,015 et 0,05 mm et plus préférentiellement entre 0,02 et 0,03 mm.

5. Lentille intracornéenne selon l'une des revendications précédentes, dans laquelle la surface avant convexe et la surface arrière concave sont reliées sur le bord extérieur du corps de base circulaire par une section intermédiaire.

6. Lentille intracornéenne selon la revendication 5, dans laquelle la zone optique intérieure (3) ne met pas de correction optique à disposition.

7. Lentille intracornéenne selon l'une des revendications 1 à 5, dans laquelle la lentille intracornéenne est une lentille diffractive.

8. Lentille intracornéenne selon l'une des revendications 1 à 5 ou 7, dans laquelle
(i) le premier rayon de courbure (Rcci) de la zone optique intérieure (3) présente une puissance optique inférieure à 1 dioptrie, et/ou
(ii) le second rayon de courbure (Rcco) de la zone optique extérieure présente une puissance optique supérieure à 1 dioptrie.

9. Lentille intracornéenne selon l'une des revendications 1 à 5 ou 7 à 8, dans laquelle la zone optique intérieure (3) présente une puissance optique d'au plus 0,5 dioptrie et la zone optique extérieure présente une puissance optique de 1,25 dioptries, et la lentille présentant un diamètre de 3,0 mm et la zone optique intérieure (3) présentant un diamètre de 1,8 mm.

10. Kit comprenant une unité de conservation (15) et une unité de pré-application (P) à l'intérieur de l'unité de conservation (15), dans lequel l'unité de conservation (15) est constituée d'un matériau étanche à l'eau et peut être fermée à l'aide d'un bouchon (16) de manière étanche à l'eau, et l'unité de pré-application (P) est équipée d'une lentille intracornéenne selon l'une des revendications 1 à 9.

11. Kit selon la revendication 10, dans lequel l'espace intérieur de l'unité de conservation (15) est rempli d'une solution saline physiologique.

12. Kit selon la revendication 10 ou 11, dans lequel l'unité de pré-application comprend (P) :
i) un boîtier (6) comportant des moyens permettant de fixer l'unité, de préférence sans rotation, à une poignée (11),
ii) un élément de réception de lentille disposé au niveau du boîtier (6) ou dans celui-ci, comprenant une section faisant saillie à partir du boîtier (6) et comportant deux unités séparées en forme de feuille (7) qui sont en contact détachable l'une avec l'autre au moins à leurs extrémités éloignées du boîtier (6) et y forment une chambre permettant la conservation d'une lentille selon l'une des revendications 1 à 9,
iii) un coulisseau (17) qui est disposé de manière mobile à l'intérieur du boîtier (6) et qui peut être déplacé entre les unités en forme de feuille (7) de l'unité de réception de lentille.

13. Kit selon la revendication 12, dans lequel les deux unités en forme de feuille (7) présentent chacune un trou (8), et sont disposées de telle manière que les trous (8) se trouvent directement l'un au-dessus de l'autre et forment une ouverture à travers le centre de la chambre permettant la conservation d'une lentille selon l'une des revendications 1 à 9.

14. Kit selon l'une des revendications 10 à 13, dans lequel une poignée (11) est en outre prévue pour la liaison à l'unité de pré-application (P).

15. Kit selon la revendication 14, dans lequel la poignée (11) peut être reliée à l'unité de pré-application (P) de telle manière que l'unité de pré-application (P) puisse être déplacée à l'intérieur de la poignée (11) à l'état relié, et que des éléments de commande (12, 13) permettant de déplacer le coulisseau (17) et l'unité de pré-application (P) restante soient prévus au niveau de la poignée (11).
